Europäisches Patentamt

European Patent Office

Office européen des brevets

(11) Publication number: **0 380 457**
**A1**

## EUROPEAN PATENT APPLICATION

(21) Application number: **90830024.7**

(51) Int. Cl.5: **A61F 5/04**

(22) Date of filing: **24.01.90**

(30) Priority: **27.01.89 IT 4758489**

(43) Date of publication of application:
**01.08.90 Bulletin 90/31**

(84) Designated Contracting States:
**BE DE ES FR GB**

(71) Applicant: **Giannetti, Donato**
**184/E Via Virgilio Melandri**
**I-00155 Roma (RM)(IT)**

(72) Inventor: **Giannetti, Donato**
**184/E Via Virgilio Melandri**
**I-00155 Roma (RM)(IT)**

(74) Representative: **Di Cerbo, Mario et al**
**Società Italiana Brevetti Piazza Poli, 42**
**I-00187 Roma RM(IT)**

(54) **Shell-like orthopedic brace.**

(57) A shell-like orthopedic brace (1), which can be prefabricated in various form and size, comprising in combination:

two halves (shells) made to the form and size of the limb to be braced;

inside each half (shell), support cushions (5) of a soft material attached to the shells and grooves (6) between said cushions;

air holes (3) placed through the body of the two shells;

means (7,8) for assuring the precise fit together of the bords of the two shells and the reversible joining together of said shells; and

means (4) for assuring the stability of the whole brace.

Fig. 1

# SHELL-LIKE ORTHOPEDIC BRACE

The invention relates to a shell-like orthopedic brace which is produced in two halves, more particularly to an orthopedic brace for curing the limbs of the human body and that of animals.

In the past when a fracture occurred, to correct the situation the bone was set and a plaster cast was applied to allow the fractured area to fuse. In this manner, a technician working with the only medium available, namely plaster, first wrapped the broken limb in a bandage, then coated the site with plaster, and waited for the plaster to dry and cure.

Over a period of time, the formation of plaster casts has become the accepted way of curing fractures and an art practiced by many in the medical field, including orthopedic doctors, technicians, and nurses, and others on the orthopedica staff. While widely practical, it is well known that, when needed for long periods of time the plaster cast is inconvenient and cumbersome. Other disadvantages of the plaster cast are that plaster casts take a long time to prepare and must be applied by an orthopedic professional. When the cast is finished, the patient is carrying and supporting the weight of a closed heavy plaster cast. Other problems arise, such as personal hygiene, physical discomfort and psychological problems.

More specifically, with the plaster cast, it is difficult to nurse the fracture with medication or to observe the curing process. Further, it is difficult for the limb to breathe in a cast because there is no circulation or air. A patient with a plaster cast is also limited as to the extent of the personal hygiene he can manage as he must be careful not to get the cast wet. The difficulty with the psychological and the physical effects of the plaster cast is frequently related to the weight of the cast, the length of time the cast is used, and the itching of the irritated areas.

Taking into consideration all of the above, one can see the advantages of the orthopedic brace fabricated in two shells the description of which follows.

The shell-like orthopedic brace of the present invention, which can be prefabricated in various form and size, comprises in combination:
two halves (shells) made to the form and size of the limb to be braced;
inside each half (shell), support cushions of a soft material attached to the shells and grooves between said cushions;
air holes placed through the body of the two shells;
means for assuring the precise fit together of the bords of the two shells and the reversible joining together of said shells; and
means for assuring the stability of the whole brace.

The orthopedic brace constructed in two halves according to the present invention in a preferred embodiment is constructed so that one half is the mirror image of the other.

The brace is preferably constructed of PVC, polyvinyl, shockproof polystyrene, or other reinforced plastic having the necessary physical properties.

The cushions and/or the grooves and/or the air holes are equal or different from each other as regards size and shape.

The cushioning (soft section) is fabricated from rubber, rubber foam, or plastic foam. The cushions can be attached to the halves by means of adhesive.

The means for assuring the precise fit together of the bords of the two shells and their reversible joining together can consist in connecting to each other holes on flanges obtained in the bords of said shells. Moreover, the means for assuring the stability of the whole brace can consist in selflocking belt means wounded round the shells or in belt means provided with reversible interlocking means.

The shell-like orthopedic brace according to the present invention can be made in a jointed version.

More precisely, the shell-like orthopedic brace for application to a specific limb of a patient, according to the present invention, comprises in combination:
a first half-shell form dimensioned to said specific limb of the patient;
a second half-shell form for cooperative functional relationship with said first half-shell form, said second half-shell form being substantially a mirror image of said first half-shell form and interconnective therewith;
each said half-shell form comprising, in turn:
one or more channel portions extending longitudinally along the shell form;
air holes positioned along said channel portions in the shell and opening therethrough;
support means of a soft material for attachment to each said half-shell form and for filling the interstice between said shell forms and said patient; and,
each said support means secured between said channel portions of the shell forms without impeding the flow of air to the limb;
whereby, upon securing said orthopedic brace to said limb of the patient, the limb is securely supported by a lightweight, stable and well-ventiled brace.

When compared to the conventional plaster cast, the orthopedic brace constructed in two halves, has more advantages like these:

Simple construction and low cost

Reduction in waiting time by 75%

Reduction in preparation time by 80%

Reduction in the number of operators

Ventilation to the injured limb through the air holes provided

Easier access to the injured limb for nursing, medical reasons, opening and closing

Re-usability of the orthopedic brace

Reduces precautions a patient must take with a cast, danger of misuse of plaster cast and psychological problems connected therewith.

As stated hereinbefore the shell-like orthopedic brace has many advantages and can be readily applied in a variety of specific limbs. Other advantages of the orthopedic brace of this invention will become apparent from the reading of the detail description which follows, especially as the brace can be constructed in various forms and measurements and can be applied by relatively non-skilled workers to the limbs of the patient.

The invention is more readily understood by viewing the figures that follow, in which similar parts are afforded the same reference numbers in the various views.

Figure 1 is a side view of the orthopedic brace.

Figure 2 is a cross-sectional view of the orthopedic brace of Figure 1, along section line II-II.

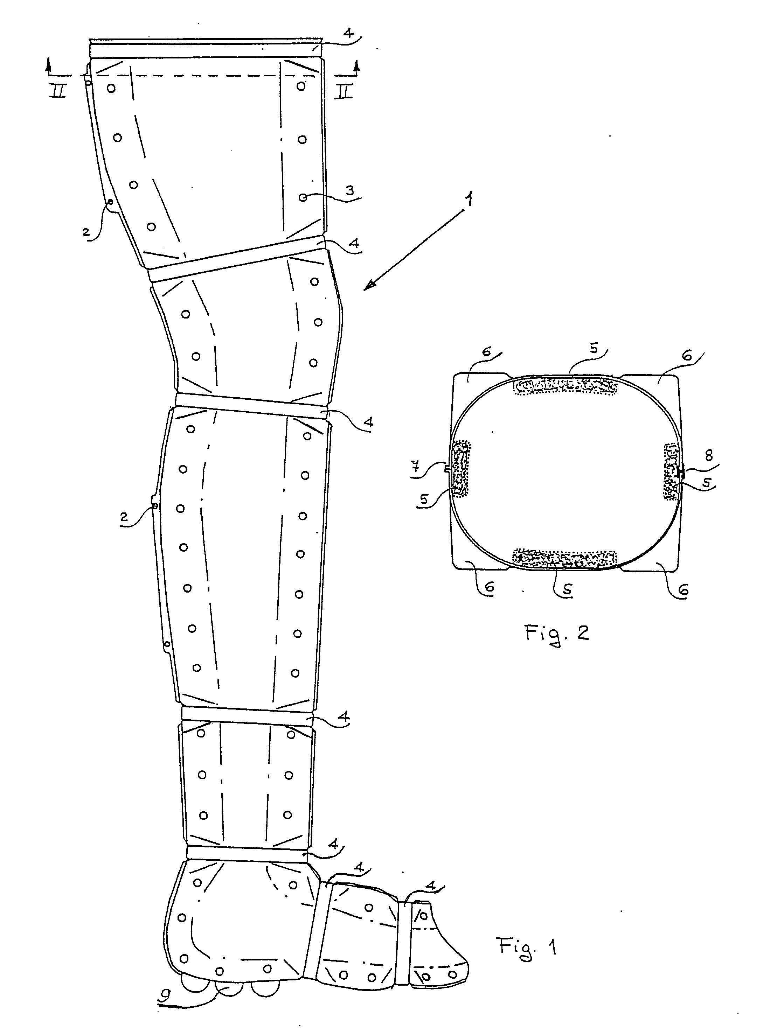

Referring to figures 1 and 2, an orthopedic brace for the human leg is shown, and referred to generally by the reference numeral 1. The orthopedic brace has an outer portion, including the bord and the flange. The orthopedic brace is structured to include two connected half shells as shown. The orthopedic brace 1 is structured to include air holes 3. The two halves are connected by belts 4. The interior of the shells are constructed to include cushions of foam rubber 5. Channels or grooves 6 provide an airway along the length of the brace. Closure devices 7 and 8 indicate respectively the system for closing the orthopedic brace and attaching the "L" or "H" construction the one to the other. Semispherical heel supports 9 are shown attached to the heel portion of the leg brace for protecting the heel from shock and improving equilibrium.

In operation, when the brace is applied to the leg and is placed to the fracture correctly, the cushions 5 give the necessary precise pressure to the injured area. It has the ridged contact with the brace as with the skin. The function of the holes 3 and the grooves 6 is to assist in a continued flow of air into the brace and ultimately the injured area.

Although the best mode of the invention has been described herein in some detail, it has not been possible to include each and every variation.

Those skilled in the art of constructing orthopedic braces will be able to make slight variations in the mechanical arrangement suggested hereby without departing from the invention and the scope of the claims appended hereto.

## Claims

1. A shell-like orthopedic brace, which can be prefabricated in various form and size, comprising in combination:

two halves (shells) made to the form and size of the limb to be braced;

inside each half (shell), support cushions of a soft material attached to the shells and grooves between said cushions;

air holes placed through the body of the two shells;

means for assuring the precise fit together of the bords of the two shells and the reversible joining together of said shells; and

means for assuring the stability of the whole brace.

2. The shell-like orthopedic brace as per claim 1, in which the two halves are mirror-image of each other.

3. The shell-like orthopedic brace as per claim 1 or 2, which is made of polyvinyl chloride.

4. The shell-like orthopedic brace as per claim 1 or 2, which is made of shockproof polystyrene.

5. The shell-like orthopedic brace as per any of the preceding claims, in which the cushions are equal or different from each other as regards size and shape.

6. The shell-like orthopedic brace as per any of the preceding claims, in which the grooves are equal or different from each other as regards size and shape.

7. The shell-like orthopedic brace as per any of the preceding claims, in which the holes are equal or different from each other as regards size and shape.

8. The shell-like orthopedic brace as per any of the preceding claims, in which the cushions are made of rubber foam and are attached to the halves by means of adhesive.

9. The shell-like orthopedic brace as per any of the preceding claims, in which the means for assuring the precise fit together of the bords of the two shells and their reversible joining together consist in connecting to each other holes on flanges obtained in the bords of said shells.

10. The shell-like orthopedic brace as per any of the preceding claims, in which the means for assuring the stability of the whole brace consist in selflocking belt means wounded round the shells or in belt means provided with reversible interlocking means.

Fig. 2

Fig. 1

## DOCUMENTS CONSIDERED TO BE RELEVANT

EP 90830024.7

| Category | Citation of document with indication, where appropriate, of relevant passages | Relevant to claim | CLASSIFICATION OF THE APPLICATION (Int. Cl.5) |
|---|---|---|---|
| A | US - A - 3 955 565 (JOHNSON) * Totality * | 1,2,5-7,10 | A 61 F 5/04 |
| A | US - A - 3 701 349 (LARSON) * Abstract; column 3, lines 17-58; column 4, lines 48-52; column 5, lines 24,25, 35-39; claims 1,2,5,6; fig. 1-4,9 * | 1,2,5-9 | |
| A | US - A - 4 727 865 (HILL-BYRNE) * Abstract; claim 1; fig. 1-4 * | 1,2,9 | |
| A | EP - A2 - 0 155 730 (GESTIONI) * Abstract; claims 1,2 ; fig. 1-4 * | 1-4,10 | |
| A | US - A - 3 831 467 (MOORE) * Abstract; column 2, lines 40-54; claim 1; fig. 1-5 * | 1,8,10 | TECHNICAL FIELDS SEARCHED (Int. Cl.5) A 61 F 5/00 |
| A | CA - A - 1 027 445 (SALVAIL) * Claim 1,2,8,9; fig. 1-4 * | 1,2,4,10 | |
| A | FR - A - 1 325 526 (J.B. THOMAS) * Claim 1; fig. 1-3 * | 1,2,10 | |

The present search report has been drawn up for all claims

| Place of search VIENNA | Date of completion of the search 26-04-1990 | Examiner TSILIDIS |
|---|---|---|